# EUROPEAN PATENT SPECIFICATION

(11) **EP 3 091 937 B1**
(45) Date of publication and mention of the grant of the patent: **20.03.2024**
(21) Application number: 15717961.5
(22) Date of filing: 09.01.2015
(51) Int. Cl.: A61F 2/01

(54) **ANATOMY INDEPENDENT DEFLECTOR**
UNABHÄNGIGER ANATOMIEDEFLEKTOR
DÉFLECTEUR INDÉPENDANT DE L'ANATOMIE

(30) Priority: 10.01.2014 US 201461926142 P
(43) Date of publication of application: 16.11.2016
(73) Proprietor: Keystone Heart Ltd., 3088900 Caesarea (IL)
(72) Inventor: SHEZIFI, Yuval, 34752 Haifa (IL); SALMAN, Iddo, 39017 Tirat Ha'carmel (IL); SHEMESH, Tzeela, Mikovski, 52295 Ramat Gan (IL)
(74) Representative: Zaboliene, Reda
(86) International application number: PCT/IB2015/000410
(87) International publication number: WO 2015/104645

(56) References cited:
- WO-A1-00/43062
- WO-A1-2014/076219
- WO-A2-2012/009558
- WO-A2-2012/085916
- SG-A1- 171 706
- US-A1- 2013 184 739
- US-B1- 6 245 087

## Description

### FIELD OF THE INVENTION

The invention relates to devices for blocking emboli in an aorta from entering arteries.

### BACKGROUND OF THE INVENTION

Devices such as intra-vascular filters may be inserted into a blood vessel prior to or during a procedure or at another time. Such devices may be inserted by way of a catheter that may be threaded through a vein or artery and into, for example, an aorta or other vessel where the device may be released from the catheter and, for example, deployed. The device may filter, deflect, or block emboli or other objects from entering into a blood supply that feeds the brain.

WO2012/085916 discloses an intra-vascular device may include a skeleton to hold a blood filter, an upper member to fit into a branch artery of an aorta, and a pair of lower members whose distal ends are not connected to each other, such lower members to press against a wall of an ascending artery and to provide lift to the device so that a middle portion of the device is above a lateral plane of the device. The device may be positioned in a middle area of an aortic arch near but not covering an opening of the branch arteries of the aorta, and may filter or deflect emboli or other large objects from entering into the branch arteries.

US 2013/0184739 discloses a device for the removal of clot obstructing the flow of blood through an arterial vessel comprises an elongate member, a clot engaging element and a capture basket.

### SUMMARY OF THE INVENTION

The invention is set out in the appended set of claims

In a first aspect, the present invention provides an intra-vascular device for deflecting emboli, the device having a lateral structure, a filter, and two wires connected to the lateral structure. The lateral structure circumscribe an elongate shape (e.g., elliptical, oval, or other non-rectilinear shape) with a length between 80 mm and 120 mm, and a width between 30 mm to 45 mm. The lateral structure be sized such that upon installation of the device in the aortic arch of a human subject, it contacts the upper walls of the aortic arch. Further, a substantially planar, elongate (e.g., elliptical, oval, or other non- rectilinear shape) filter be attached to the length and width of the lateral structure. The first wire be attached to the distal end of the lateral structure, and the second wire be attached to the proximal end of the lateral structure.

In additional embodiments, the lateral structure of the device forms a seal with the walls of the aortic arch, upon installation of the device in the aortic arch. In some embodiments, the filter of the device extends beyond the perimeter circumscribed by the lateral structure, e.g., to form a seal against the blood vessel wall, e.g., aortic arch.

The pores of the filters of the above-devices can be sized such that they are permeable to blood, (e.g., red blood cells (erythrocytes), white blood cells (leukocytes), platelets (thrombocytes), and plasma) but not significantly permeable to emboli, e.g., having a dimension of greater than 50, 100, or 200 µm.

In certain embodiments, the two wires of the device can have the same or differing stiffness (e.g., the first wire can be stiffer than the second, or the second wire can be stiffer than the first) and can be readily malleable. The device can be made from metal, e.g., Nitinol wire. The diameter of the wires and lateral structure of the device can be 1, 0.5, 0.25, 0.1 mm, or less. The lateral structure and/or a first and/or a second wire can be formed by twisting two or more wires together to form a composite wire with the desired thickness (e.g., having a diameter less than about 0.5 mm) and stiffness.

In certain embodiments, a first and/or a second wire is attached to the filter or lateral structure along the length of the lateral structure (e.g., along a midline of the lateral structure). In certain embodiments, the second wire is a hollow tube.

In one embodiment, the first wire of the device has a screw-like mechanism positioned at its distal end, and the filter or lateral structure of the device includes a mating component at its distal end that is suitable to receive the screw-like mechanism. The mating component of the filter or lateral structure of the device may include a bead, e.g., a spherical bead, attached near the distal end of the filter, or additional elements to irreversibly or reversibly connect the lateral structure or filter to the first or second wires (e.g., a screw and recess to accept the screw or a ball and snare mechanism). The additional elements may include one or more (e.g., 2, 3, 4, or more) ball(s), e.g., spherical ball(s), or tip(s) at the distal and proximal ends of the lateral structure. A first and/or second wire of the device may also include a snare that may join with a bead, ball, tip, or other receiving element attached to the filter or lateral structure of the device.

Any of the above embodiments, the devices of the invention can further include radiopaque markers.

In another aspect, the invention features a system for deflecting emboli, which includes any of the aforementioned embodiments of an intra-vascular device having a lateral structure, a filter, and first and second wires; and a catheter, into which the device can be loaded. In some embodiments, the catheter of the system contains a guide wire.

The above systems can further include a protected lip at the distal end of the system. The protected lip can be a dilator (e.g., with a diameter smaller than the catheter), which is positioned distal to the catheter. The systems of the invention may also include a pigtail catheter.

In one embodiment of the system of the invention, when loaded into the catheter, the lateral structure and filter of the device can be compressed, e.g., and positioned behind a dilator, if present. In another embodiment, the lateral structure and filter of the device expands upon retraction of the catheter relative to the device (or advancement of the device relative to the catheter). The catheters described above can have a distal opening that is perpendicular to the length of the catheter or non-perpendicular to the length of the catheter. The angle of the opening can be adjusted to permit delivery of the device in a desired orientation.

In another aspect, the description features methods, which are not according to the invention and are present for illustration purposes only, of introducing any of the above devices into a subject by inserting into a blood vessel (e.g., the aortic arch) of the subject a catheter containing the device (the device can be loaded into the catheter prior to or after introduction of the catheter into the subject). The device can be loaded into the catheter by applying force to, e.g., the first wire. Upon retraction of the catheter relative to the device (or advancement of the device relative to the catheter), the lateral structure and filter deploy into the blood vessel at a desired location, for example, into the aortic arch. Furthermore, upon installation in an aortic arch, the lateral structure and/or filter of the above devices can be capable of forming a seal with the walls of the blood vessel (e.g., aortic arch). The device can be positioned within the aortic arch by antagonistically pushing and pulling on the first and the second wires.

In certain embodiments, the device is loaded into a catheter prior to, or after introduction of the catheter into a subject. The catheter can further include a guidewire. A pigtail catheter may also be introduced through the catheter. Further, the pigtail catheter may be inserted over a guidewire that may be retracted through the catheter prior to deployment of the device.

As used herein, the term "substantially planar" refers to a radius of curvature of no more than 80 mm (e.g., no more than 10 mm, 20 mm, 30 mm, 40 mm, 50 mm, 60 mm, or 70 mm).

As used herein, the term "elongate" refers to a shape having a length that is greater than its width. As used herein, the term "provide structural support" refers to the property contributing to shape and stiffness of the device.

As used herein, the term "wires" refers to any elongated structure (e.g., cords, fibers, yarns, filaments, cables, and threads) fabricated from any non-degradable material (e.g., polycarbonate, polytetrafluorothylene (PTFE), expanded polytetrafluorothylene (ePTFE), polyvinylidene fluoride (PVDF), polypropylene, porous urethane, metal, Nitinol, fluoropolymers (e.g., Teflon^{®}), cobalt chromium alloys (CoCr), and para-aramid (Kevlar^{®}), or textile (e.g., nylon, polyester (e.g., Dacron^{®}), or silk).

As used herein, the term "pigtail catheter" refers to a surgical device that is used to introduce radio-opaque contrast for implant (e.g., a prosthetic aortic valve) confirmation.

As used herein, the term "dilator" refers to a surgical instrument that is used to make a bodily orifice, such as a blood vessel, become wider, larger, or more open.

### BRIEF DESCRIPTION OF THE DRAW INGS

Fig. 1 A shows an intra-vascular device deployed from a catheter and connected to a dilator. Fig. 1B shows a dilator being withdrawn (e.g., from a blood vessel) via a catheter. Fig. 1C shows the device deployed from a catheter, in the presence of a pigtail catheter, in accordance with an embodiment of the invention.
Fig. 2A is a view of an intra-vascular device deployed from a catheter. Fig. 2B is a top-view of a circular ring frame. Fig. 2C is a top-view of an elongate filter. Fig. 2D is a cross-sectional view of the elongate filter installed within a blood vessel, in accordance with an embodiment of the invention.
Fig. 3A is a view of an intra-vascular device having two wires attached to it. Fig. 3B is a side- view of the device with the two wires still attached while positioned in a blood vessel. Fig. 3C is a side- view of the device being deflected by the two wires while still positioned in a blood vessel, in accordance with an embodiment of the invention.
Fig. 4 is a schematic illustration of an intra-vascular device having a screw mating bead mechanism in accordance with an embodiment of the invention.
Fig. 5 is a view of an intra-vascular device deployed from a catheter with an opening that is not perpendicular to the longitudinal axis of the catheter in accordance with an embodiment of the invention.
Figs. 6A-C are schematic diagrams of an intra-vascular device having a wire attached to the filter of the device, within the plane of the filter in accordance with various embodiments of the invention.
Figs. 7A-D are schematic illustrations of an intra-vascular device with a non-symmetric design being deployed from a catheter in accordance with an embodiment of the invention.
Figs. 8A-B are schematic illustrations of an intra-vascular device with a symmetric design in accordance with an embodiment of the invention.

### DETAILED DESCRIPTION OF THE PREFERRED EMBODIMENTS

The present invention relates to methods of inhibiting the potentially harmful passage of particulates through the blood stream. Particulates that may be present in blood include, without limitation, blood clots, calcified debris, and emboli. While extremely small particulates may not cause significant harm, passage of larger particulates can result in stroke or other adverse outcomes. The risk of damage resulting from the passage of particulates can increase in association with certain conditions or medical procedures that perturb the vasculature. In order to moderate these risks, the invention features an intra-vascular device having a filter which is used to prevent particles in a blood vessel from passing through the filter, a lateral structure to support the filter, and two wires attached the lateral structure, one each at its distal and proximal ends. The lateral structure is substantially planar and does not include any supporting members that extend above or below the plane of the filter. In preferred embodiments, the two wires attached to the ends of the lateral structure may be used to deflect or control the orientation of the filter, after it has already been positioned within a blood vessel (e.g., the aortic arch). The two wires can be capable of readily moving through a delivery system to deliver the device to a desired location, and they can be pushed and pulled antagonistically to deflect the device.

In some embodiments, after the filter has been installed within the blood vessel and oriented as desired via use of the two wires, these wires may then be detached from the device leaving behind only the filter and its lateral structure in the blood vessel. This detachment may be facilitated by a screw-like mechanism attached to the distal end of a first wire of the device that is suitably mated to an element attached to the distal end of the filter, lateral structure, or another part of the device. The mating element attached to the device capable for conjoining the screw, or a suitable alternative, may be a bead.

In another embodiment, the device features a small ball or tip attached to the lateral structure at both its proximal and distal ends. Such an embodiment allows for attachment of a first and/or second wire to the lateral structure via a snare positioned around each small ball or tip. The snare, when attached to the tip or the ball, allows for deflection or control of the orientation of the device by pushing and pulling on the first and second wires. One, or both, of the wires may be detached from the lateral structure via the removal of the snares from around the attached ball or tip. These additional features of the device may remediate potential entanglement between the first wire and any instrumentation that may be passed between the first wire and the filter, for instance.

One of the wires of the device may be attached or sewn onto the filter or the lateral structure within the same plane or the filter or the lateral structure. For example, the wire may be attached along the middle of the filter and allow for bending of the first wire and filter into a concave shape prior to use. The bent wire may be rigid and attached to the filter, for instance, on its upper side. The filter may alternatively have a hollow tube, made of a stable, flexible material, positioned and attached along the length (e.g., midline) of the filter. The hollow tube may be the second wire of the device. In some embodiments, the tube has a first wire passing through it, capable of moving back and forth within the tube, which results in the overall deflection of the tube and as such, a change in the shape of the filter. Such a tube may include two stoppers, one positioned at each end, to contain and control the movement of the wire within the tube.

The device may have a non-symmetric design with respect to the orientation of the wires to the lateral structure. In an alternative embodiment, the device has a symmetric design, which allows for the filter to be oriented in an upright, concave manner or an inverted, convex manner. The device may also feature one or more ends that may be thinner or narrower than the rest of the device. The narrowing of the ends of the device may terminate in a rounded knob shape but not a point.

The lateral structure of the device provides, e.g., structure to the filter, and can facilitate control of the filter within the anatomy of the primary blood vessel. For example, this lateral structure can permit an operator to control the orientation of the device within the primary blood vessel to press the device against certain features of the primary blood vessel (e.g., to press the device against the orifice of one or more secondary blood vessels or against the walls of the primary blood vessel). The devices of the invention may feature a lateral structure that is a spring ring wire frame made of, for example, a flexible material. This spring ring frame can be shaped into an oval or elliptical shape. In some embodiments, the devices of the invention can also feature an outer lateral structure connected to an internal lateral structure to form a skeleton with, for example, an elliptical shape. The outer lateral structure can provide additional structural support for the device and can facilitate the creation of a seal between the filter of the device and a blood vessel wall. Alternatively, the filter itself may create a seal against the blood vessel wall by extending beyond the perimeter of the lateral structure.

The device of the invention may include a filter, a lateral structure with no supporting members extending beyond its plane, and two wires. The device can filter and/or deflect emboli or other large objects from entering protected secondary vessels. The device may be capable of collapse along its longitudinal axis for ease of delivery to the treatment site. The device may further be compatible with common delivery methods used in interventional cardiology (e.g., TAVI procedures). The device may be integrated into a delivery, e.g., a catheter, system. In some embodiments, the device may be detachable from the delivery wires. Upon retrieval, the device may be retracted in an orientation substantially similar to the original deployment orientation.

Any of the lateral structures or delivery wires of the devices of the invention can be fabricated in whole or in part from, e.g., Nitinol or other metal wire, superelastic or shape memory alloy material, readily malleable material, or nylon. The metal wire may include, e.g., tantalum or platinum. The diameter of the wire of the devices may be less than or equal to 1.0 mm (e.g., 1.0, 0.5, 0.25, 0.1 mm, or less). The thickness or diameter of the wire of the lateral structures and/or delivery wires may have different thicknesses to confer differing properties such as rigidity or flexibility. For instance, the lateral structures of the devices can be stiffened by the inclusion of one or more (e.g., 1, 2, 3, 4, 5, or more) twists of the wire. Furthermore, multiple wires of a certain gauge can be wound together to increase the stiffness of the lateral structure (e.g., the lateral structure can include 2, 3, 4, 5, or more wires of to increase the stiffness of the intra-vascular device),

The filters of the intra-vascular device of the invention can include a mesh (e.g., a mesh fabricated with Nitinol or other metal wire, nylon, or a combination of both) or perforated film. In devices where a mesh is present, the filter mesh can be rectilinear (e.g., square) or rhomboid. In devices where the pores of the filter are rectilinear or rhomboid, one or both lateral dimensions of the pore can be between 50 and 1000 microns (e.g., 100, 200, 300, 400, 500, 600, or more microns). When a perforated film is present, the pores formed in the perforated film include a varied or unvaried shape, have a varied or constant density across the film, and/or have a constant or varied size. The size of the pores of the filter allows the passage of blood cells (e.g., red blood cells (erythrocytes), white blood cells (leukocytes), platelets (thrombocytes), and plasma), while being impermeable to emboli or other undesirable particles larger than the pore dimensions. Emboli filtered by the filter of the present invention are typically emboli or other endothelial particles larger in one or more dimensions than an aperture of the mesh of the filter. Emboli filtered by the intra-vascular device of the present invention may be sized to have a dimension greater than 50 µm, e.g., 50 µm, 60 µm, 70 µm, 80 µm, 90 µm, 100 µm, 200 µm, 300 µm, 400 µm, 500 µm, or 1000 µm or more. In various embodiments, it is desirable to track the progress of all or a portion of the device of the present invention or of a treatment apparatus used in conjunction with the device of the present invention. A variety of mechanisms for tracking the progress of all or a portion of a device, e.g., by visualizing progress, are contemplated. Methods of tracking include, without limitation, X-ray, fluoroscopy, ultrasound, echocardiography, MRI (magnetic resonance imaging), direct angioscopy, near infrared angiology, intra-vascular ultrasound, CT (computerized tomography) scan, and/or any other suitable imaging technology.

In certain instances, a device may require one or more modifications to facilitate one or more methods of tracking the progress of all or a portion of the device. In particular embodiments, one or more radiopaque elements are attached to, included in, or integrated with the device. For example, portions of the lateral structure or filter can be constructed out of Drawn Filled Tubing (DFT wire). Such wire can contain a core of tantalum and/or platinum and an outer material of Nitinol. In certain embodiments, the DFT wire can be incorporated into all or a portion of the intra-vascular device lateral structure, wires, or filter. In embodiments where radiopaque wire (e.g., DFT wire) is used in the filter, it can be used throughout the filter or in a certain subset thereof.

In particular embodiments, including some in which multiple radiopaque elements are attached to, included in, or integrated with a device, it is possible to detect both the progress and particular orientation of all or a portion of a device. In still more particular embodiments, a plurality of radiopaque elements are attached to, included in, or integrated with the filter in a manner that is irregular in two or three dimensions of one or more conformations of the filter, such that the location, orientation, and/or conformation of the filter is indicated upon detection of the radiopaque elements.

The description also features methods, which are not according to the invention and are present for illustration purposes only, of use of the intra-vascular devices having a filter which is used to prevent particles in a blood vessel from passing through the filter, a lateral structure to support the filter, and two wires attached the lateral structure, one each at its distal and proximal ends. The devices of the invention may be inserted into an aortic arch of a subject. The length of the device may be from approximately 80 mm to 120 mm, or otherwise as may be necessary to approximate a distance between an upper wall of an ascending aorta of a subject, upstream of an opening of an innominate artery and at an upper wall of a descending aorta of a subject downstream of an opening of a left subclavian artery. The width of the device may be from 30 mm to 45 mm or otherwise as may approximate an internal diameter of an aorta of a subject. The device may be introduced into a blood vessel of a subject in a collapsed form and contained within a catheter. A pigtail catheter may also be introduced through the catheter to enable visualization and positioning of the device. The pigtail catheter may be inserted over a guide wire, which can be subsequently retracted through the catheter before the device is deployed. The first wire of the device may be utilized to advance the device through the catheter. Upon reaching the desired location within the blood vessel of a subject, the catheter may be retracted, enabling the lateral structure and filter of the device to assume an extended form upon its release or deployment from the catheter. The position of the device in the desired location, such as the aortic arch, can be adjusted by antagonistically pushing and pulling of the first and second wires of the device. In the desired position, the device may extend the filter attached to the lateral structure so that the filter assumes a position approximately midway between an upper wall of the aortic arch and a lower wall of the aortic arch, and extends over the distance between the branch arteries of the aorta. The positioned device filters embolic material from entering the branch arteries of the aorta. In one embodiment, a device according to an embodiment of the present invention can be used for protection of the brain from emboli prior to, during, and/or after an invasive intracardiac procedure, such as balloon aortic valvuloplasty, balloon mitral valvuloplsty, electrophysiological studies, with or without ablation of ectopic rhythmic sites, insertion of automatic defibrillators, percutaneous valve repair or replacement, or other procedures. Embodiments of the device can be used, for example, in subjects with severe aortic atheroma for brain protection during routine heart catheterization, or for endovascular "cleaning" of atheromatous or thrombotic material. Such an embodiment could be used in subjects with high risk or propensity to form intracardiac clots, for example subjects with hematological disease, arrhythmia of the heart, artificial heart subjects, assist-device subjects, mechanical valve replacement subjects, subjects following intracardiac repair of a pathology, or subjects with congenital heart disease such as patent foramen ovale, and so forth. Other applications of blood particulate filters, medical procedures that benefit from the use of blood particulate filters, and patients at risk of damage resulting from blood particulates are known in the art.

A device according to an embodiment of the present invention can be used, for example, temporarily for acute conditions. For example, the device can be inserted for the duration, or the known duration, of the condition. For example, the device may be inserted temporarily to protect against cardio embolic stroke or embolic stroke. The device of the present invention may be used to reduce the risk of damage resulting from blood particulates, such as emboli in subjects from suffering conditions associated with an elevated risk thereof, such as acute myocardial infarction (AM I). Thus, in further embodiments, the device may be inserted for the duration of a procedure or treatment. In other embodiments, the device may be inserted for a long-term period, or permanently.

One particular use or outcome of the use, of many embodiments of the present invention includes the prevention of particulates from reaching the brain or preventing blood flow to the brain.

A device of the present invention may be used in conjunction with one or more pharmaceutical compositions, such as a drug known to treat endocarditis or blood clots.

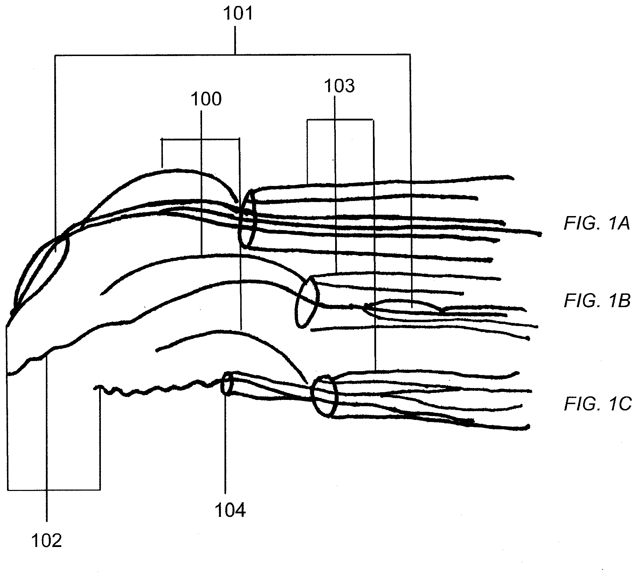

Reference is made to Figures 1 A-1 C, which depict delivery systems of the invention. Fig. 1A which shows an intra-vascular device (IVD) 100 deployed from a catheter and connected to a dilator. Fig. 1B shows a dilator 101 being withdrawn (e.g., from a blood vessel) relative to IVD 100 via a catheter 103. Fig. 1C shows the device deployed from a catheter 103, in the presence of a pigtail catheter 104, in accordance with an embodiment of the invention. The IVD 1 00 may be inserted into, for example, a blood vessel, and may prevent the passage of or block or filter out particles, such as, blood clots, embolisms, or other objects that may damage a blood vessel into which such particles may lodge. IVD 100 may be inserted into a vessel by way of, for example, a catheter 103, and may be threaded into, for example, a blood vessel into which IVD 100 may be implanted. Catheter 103 may be advanced over guide wire 102. In order to readily advance through, for example, a blood vessel, the catheter 103 may need to have a protected lip. This may be achieved by using a catheter with, for example, a dilator 101 which may smooth the passage of the catheter 103 through the blood vessel.

In some embodiments, the diameter of the dilator 101 positioned within catheter 103 may be sufficiently small such that the guide wire 102 may move past the dilator 101, when both are simultaneously present in the catheter. Once the catheter 103 is partially withdrawn, the IVD 100 may unfold, and the dilator 101 may be withdrawn via the catheter 103. The dilator 101 may be sufficiently flexible to be withdrawn via the catheter 103 despite the continued presence of, for example, various wires within the same catheter 103. In some embodiments, there is sufficient space for a pig tail catheter 104 to be inserted over the guide wire 102, via the catheter 103, into the ascending aorta. Other methods of implanting IVD 100 into a blood vessel are possible.

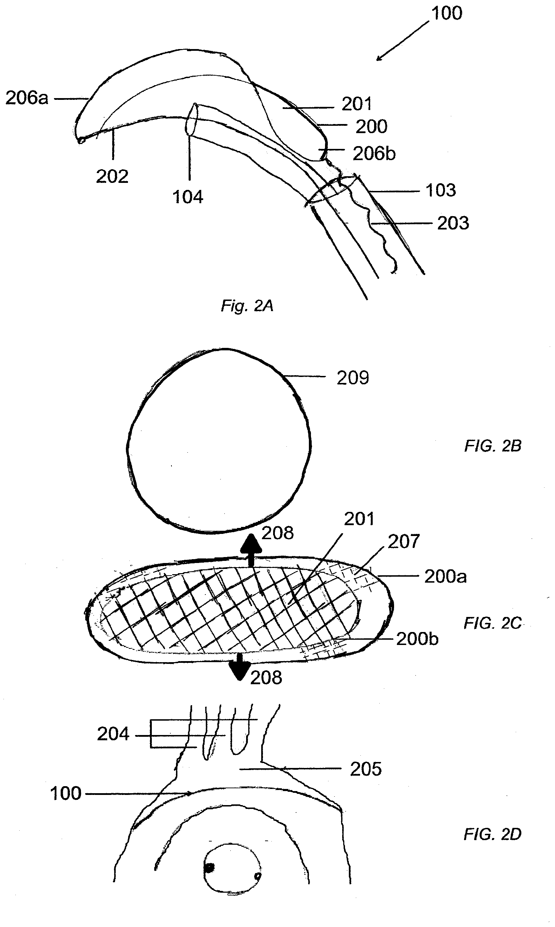

Reference is made to Fig. 2A, a view of an intra-vascular device 100 deployed from a catheter 103. Wire 203 may be collapsible, and wire 202 may be stiff Fig. 2B is a top-view of a circular ring frame 209. Fig. 2C is a top-view of an elongate filter. Fig. 2D is a cross-sectional view of the elongate filter installed within a blood vessel, all in accordance with an embodiment of the invention. IVD 1 00 may include a lateral structure 200, a filter 201 that may be held or supported by the lateral structure 200. One or more ends 206a and 206b of IVD 100 may be thinner or narrower than the rest of IVD 100. In some embodiments, an end 206 may terminate in a rounded knob shape, so that, for example, IVD 100 narrows but does not terminate in a point.

In some embodiments, lateral structure 200 may include, or be constructed of Nitinol or other superelastic or shape memory alloy or material. Other materials may be used to make the lateral structure and the skeleton. Fig. 2C is a schematic of the IVD 1 00 with an outer 200a and inner 200b structure that forms a skeleton 207, which may improve apposition between the filter 201 and, for example, the upper walls of the aortic arch 205. Filter 201 may be, or include, perforated film or a fine wire netting or mesh, such as a mesh having holes or eyes of 300 microns or less such that, for example, particles that are larger than 300 microns are prevented from passing through the filter. Other sizes of holes or eyes may be used. In some embodiments, a shape of the filter may be defined or supported by the shape of the lateral structure 200. IVD 100 may assume a shape of an extended oval or willow leaf. Other shapes may also be used.

In some embodiments, a circular, spring ring wire frame 209 may be made into inner lateral structure 200b having an elongate shape, which may form the part of skeleton 207 of IVD 100. The inner lateral structure 200b may exert a residual outward force 208 which may help it position itself in, for example, an aortic arch 205 and may help prevent particles such as, emboli, from entering the carotid branches 204. The filter 201 may or may not be substantially planar. As depicted in Fig. 2D, upon installation in an aortic arch 205, IVD 100 may only contact the upper wall of an ascending aorta and the upper wall of a descending aorta. IVD 100 may or may not also contact the orifices of the carotid branches 204.

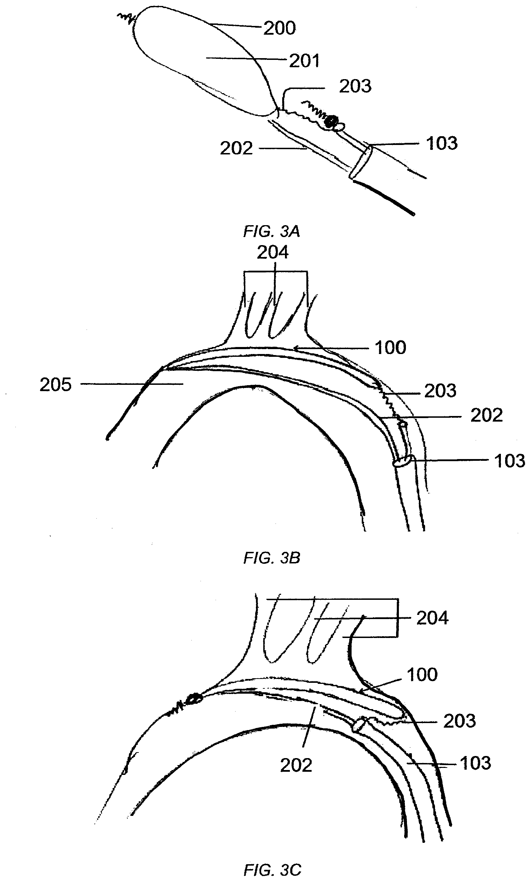

Reference is made to Fig. 3A, which is a view of an intra-vascular device 100 having two wires (202, 203) attached to the filter 201, Fig. 3B, which is a side-view of the device 100 positioned in an aortic arch 205 and Fig. 3C, which is a side-view of the device 100 being manipulated by the two wires (202, 203). In some embodiments, IVD 100 is introduced by, for example, a catheter 103 into an aortic arch 205 or other vessel. IVD 100 may be implanted so that its lateral structure 200 extends from an area near, or touching, a medial wall of the ascending aorta to an area near or touching a medial wall of a descending aorta as depicted in Figs. 3B and 3C. Other implant positions are possible. In a preferred embodiment, the filter 201 covers one or more of an opening of a brachiocephalic, left common carotid, left subclavian artery, or other blood vessel. In some embodiments, filtering particles from entering such arteries may prevent particles from entering a brain of a patient and may direct particles to a lower portion of a body where such particles may be less damaging.

In some embodiments, a first wire 202 is attached to the distal end of the device's lateral structure 200 and a second wire 203 is attached to the proximal end of the lateral structure. In some embodiments, the first wire 202 is stiffer than the second wire 203 and upon installation of the device in, for example, an aortic arch 205, these wires (202, 203) may be pushed and pulled antagonistically to deflect IVD 100. These wires (202, 203) may be used to deliver the device to a given location within, for example, a blood vessel and may also be capable of readily moving through a catheter 103.

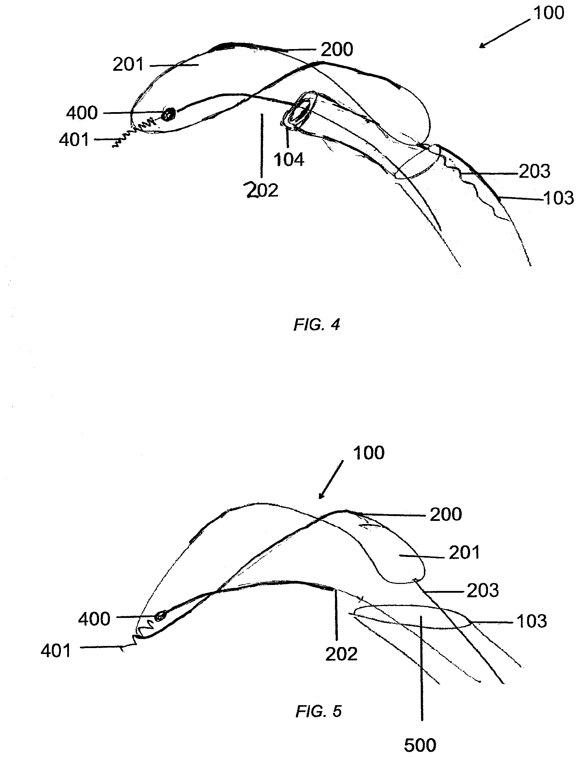

Reference is made to Fig. 4, a schematic illustration of an IVD 100 having a bead mechanism 400 in accordance with an embodiment of the invention. The IVD 100 may have a small bead 400 attached to it which can be suited for conjoining with a screw. The bead 400 can also be suitable for decreasing the likelihood of the device resulting in trauma to the artery. This element that is capable of receiving and mating with a screw may or may not be a bead. Some embodiments have a screw-like mechanism 401, located at the distal extremity of the first wire 202. The first wire 202 may be stiff, and the second wire 203 may be collapsible or stiff. In some embodiments, the bead 400 capable of conjoining with a screw, or a suitable alternative, is attached to the filter 201, lateral structure 200, or another part of the device. In some embodiments, these two mechanisms (400, 401) are utilized in attaching the first wire 202 to the lateral structure 200, filter 201, or another part of the device, via the conjoining of the screw-like mechanism 401 and the bead 400. Conversely, the first wire 202 may be detached from the lateral structure 200 through the removal of the screw-like mechanism 401 from the bead 400, or its alternative. The same arrangement of the screw-like mechanism 401 and bead 400, or its suitable alternative, may apply to the second wire 203 of the IVD 100. In some embodiments, the element of the device receiving the screw is not a bead. These features may, for example, remediate potential entanglement between the first wire 202 and any instrumentation that may, for example, pass between the first wire 202 and the filter 201.

In some embodiments, both the first wire and second wire (202, 203) are of equal stiffness. In some embodiments, the guide wire 102 passes through the filter 201 at a distal position. In some embodiments, the catheter 103 presses against the guide wire's (102) passage way at the lateral structure 200 of the filter 201, which may cause the guide wire 102 to release itself from the filter 201 allowing, for example, the catheter 103 to advance further down the blood vessel.

In some embodiments, the end of the catheter 103 positioned inside, for example, a blood vessel, has an opening that is not perpendicular to the longitudinal axis of the catheter 103. Reference is made to Fig. 5, a view of an intra-vascular device 100 deployed from a catheter 103 with a non-right angle opening 500 in accordance with an embodiment of the invention. In this embodiment, the angle of opening 500 results in a substantial amount of the opening 500 being positioned beneath the plane of the filter 201.

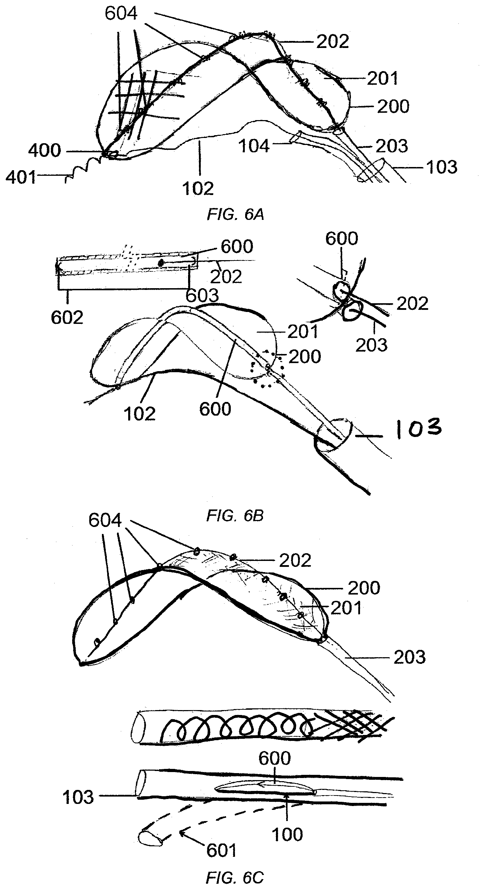

Reference is made to Figs. 6A-C, which are schematic diagrams of an intra-vascular device 100 having a wire 202 attached at attachment points 604 to the filter 201 of the device, within the plane of the filter 201 in accordance with several embodiments of the invention. In some embodiments, the first wire 202 is attached to the IVD 100, for example, by sewing it to the filter 201 at various attachment points 604 along the filter. The first wire 202 may be bent prior to its use, for example, into a concave shape, which may result in the filter 201 bending into a similar shape. In these embodiments, the guide wire 102 is still present and may pass through, or be connected to, the filter 201 at a position in its distal vicinity. As shown in Fig. 6A, the second wire 203 may be a tube through which the first wire passes. The filter 201 may have a flexible, hollow tube 600 attached to, and positioned, in the middle of the filter 201. The tube 600 may be positioned along the length of the filter 201. The tube 600 may be made of any stable, flexible material and may have two stoppers 602 and 603 positioned at either of its two ends. This embodiment may be arranged such that the first wire 202 is capable of moving back and forth within the tube 600, resulting in its overall deflection and a change of shape of the filter 201 as depicted in Fig. 6B. Further, these embodiments also remediate potential entanglement between the guide wire 102 and other wires. In some embodiments, the bent first wire 202, when attached to the filter, is attached specifically to the upper side of the filter and may be rigid. As depicted in Fig. 6C, the curvature of this first wire 202 may result in the mono-lateral deflection 601 of the related catheter 103 away from its longitudinal axis, while the first wire 202 is advancing through the catheter 103. In some of these embodiments, a guide wire 102 is layered throughout the catheter 103 in advance to, for example, assist the advancement of a pigtail catheter 104 through it as depicted in Fig. 6A. In some embodiments, the pigtail catheter 104 is used to connect various elements of the device together, such as the first wire 202 and the filter 201. In some embodiments, the second wire 203 is hollow, and the first wire 202 passes through the hollow second wire 203.

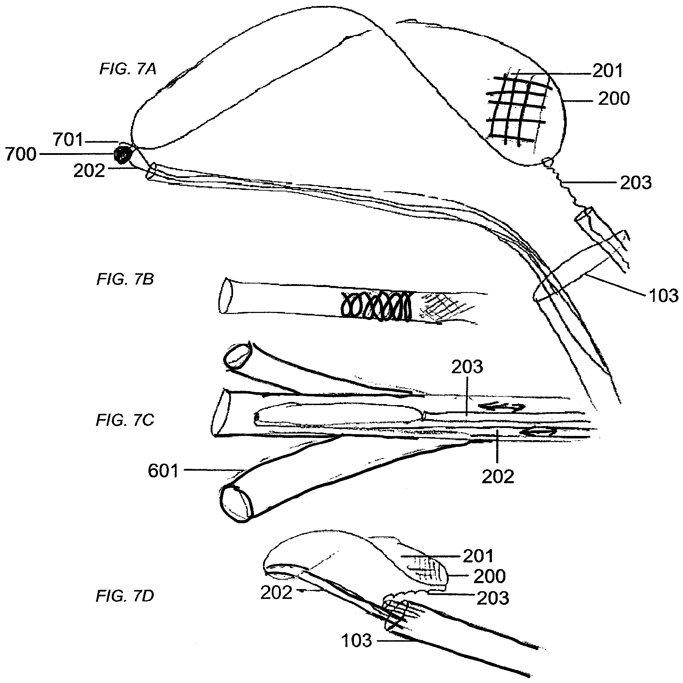

Reference is made to Figs.7A-D, schematic illustrations of an intra-vascular device 100 with a non-symmetric design in accordance with an embodiment of the invention. Fig. 7A depicts an intra-vascular device 100 with a non-symmetric design and detachable or partially detachable wires (202, 203) at both ends. In some embodiments, the lateral structure 200 of IVD 100 has a small ball or tip (700) attached to both its distal and proximal ends. The first and/or second wires (202, 203) may be attached to the lateral structure 200 via a snare 701 positioned around each small ball 700. In some embodiments, the snare 701, when attached to the tip 700, still allows for useful deflecting or controlling of the orientation of IVD 1 00 by the first and second wires (202, 203). One, or both, of the wires (202, 203) may be detached from the lateral structure 200 via the removal of the snares 701 from around the attached ball 700. Further, even when connected to the lateral structure 200 by a snare 701, the first and second wires (202, 203) may still be used for pushing and pulling in order to deflect or re-orient the IVD 100. In some embodiments, a torquable catheter in which the distal end is designed to prevent the formation of kinks or sharp twists (as depicted in Figs. 7B and 7C), is used to advance IVD 100. Catheters such as these may experience bilateral deflection when there is an IVD 100 positioned inside, due to, for example, the pushing and pulling of the first and second wires (202, 203).

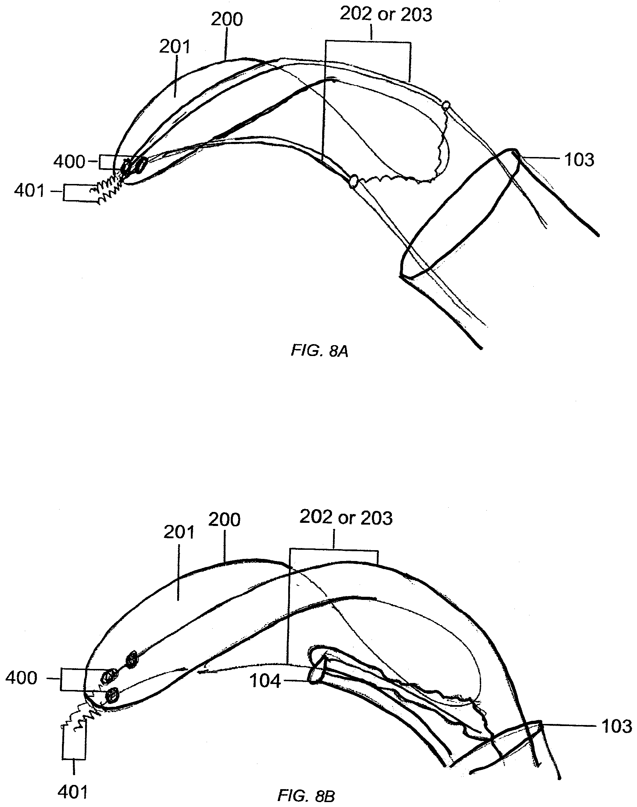

Reference is made to Figs. 8A-B, which are schematic illustrations of an intra-vascular device 100 with a symmetric design in accordance with an embodiment of the invention. In some embodiments, the symmetric design allows the filter 201 to be oriented, for example, in an upright, concave manner. In some embodiments, the symmetric design allows the filter 201 to be oriented, for example, in an inverted, convex manner. These different orientations are depicted superimposed onto each other in Figs. 8A and 8B. Further, the symmetric design of the device may allow for its upright or inverse positioning upon its installation in a blood vessel. The second wire 203 may also be removed if it becomes entangled. In some embodiments, the rigidity, or lack thereof, of the lower positioned wire of the two otherwise equivalent wires (202, 203), determines how much the catheter 103 is capable of advancing forward so that it is positioned beneath the filter 201. In some embodiments, the first and/or the second wire (202, 203) may both be attached to filter 201 at its distal and proximal ends in a relatively symmetrical manner.

There may be multiple ways of connecting the first and/or second wires (202, 203) to the filter 201 of the IVD 100. In some embodiments, a snare 701 is positioned at the end of the first and/or second wires (202, 203). This snare 701 may be looped onto a tip 700 at the ends of the frame 200 of the filter, allowing the wires (202, 203) to be attached and detached from the filter 201 accordingly. In some embodiments, the first and/or second wires (202, 203) include a screw-like mechanism 401 at their distal extremity. This screw-like mechanism 401 may be able to receivingly conjoin with suitable elements, such as a bead 400 situated on the lateral structure 200, filter 201, or another part of the device. Through screwing and unscrewing the first and/or second wires (202, 203) may be attached or detached respectively, from the rest of IVD 100. Other methods of attaching and detaching the first and/or second wires to IVD 100 may be possible.

In other examples, IVD (100) is adapted for use with other embolism protection devices (e.g., those described U.S Application Nos. 13/300,936, and 13/205,255; in U.S. Publication Nos. 2008-0255603 and 2011-0106137; and in U.S. Patent Nos. 8,062,324 and 7,232,453).

Although the foregoing invention has been described in some detail by way of illustration and example for purposes of clarity of understanding, it will be readily apparent to those of ordinary skill in the art in light of the teachings of this invention that certain changes and modifications may be made thereto without departing from the scope of the appended claims.

## Claims

1. A device for deflecting emboli from entering secondary vessels, the device comprising:
a lateral structure (200) made from a spring ring wire circumscribing an elongate shape,;
a substantially planar, elongate filter (201) attached to the length and width of said lateral structure (200); wherein said lateral structure is sized such that upon installation of said device in an aortic arch of a human subject, said device contacts the upper walls of said aortic arch;
**characterized in that**:
a first wire (202) attached to the distal end of said lateral structure; and
a second wire (203) attached to the proximal end of said lateral structure wherein said first and second wires are configured for delivering said device to said aortic arch.

2. The device as in claim 1, wherein said first wire (202) further comprises a screw-like mechanism (401) positioned at its distal end and said filter (201) or said lateral structure (200) comprises a mating component at its distal end suitable to receive said screw-like mechanism (401).

3. The device as in claim 2, wherein said mating component comprises a bead (400) attached at the distal end of said filter (201) or said lateral structure (200).

4. The device as in any one of claims 1-3, wherein said filter (201) extends beyond the perimeter circumscribed by said lateral structure (200); or wherein said first wire (202) is stiffer than said second wire (203), or said second wire (203) is stiffer than said first wire (202); or wherein said first wire (202) and said second wire (203) are of equal stiffness.

5. The device as in any one of claims 1-4, wherein said device is made from metal or polymer wire; or wherein said device is made from Nitinol wire.

6. The device as in any one of claims 1-5, wherein the diameter of said wire is about 1.0 mm or less.

7. The device as in claim 6, wherein said lateral structure (200) comprises two or more twists of said wire having a diameter less than about 1.0 mm

8. The device as in any one of claims 1-7, wherein said device further comprises radiopaque markers; and/or wherein said filter (201) is not significantly permeable to emboli having a dimension of greater than 50, 100, or 200 µm; and/or wherein said first (202) or second (203) wire comprises readily malleable wire.

9. The device as in any one of claims 1-8, wherein said second wire (203) is a hollow tube (600); and/or wherein said lateral structure (200) further comprises two balls or tips (700) at said distal and proximal ends; and/or wherein said first wire (202) and/or said second wire (202) includes a snare (701), and said proximal and/or distal end of said filter (201) includes a bead (400), or other receiving element, that joins with said snare (701); and/or wherein said filter (201) further comprises a hollow tube (600)positioned along the length of said filter.

10. A system comprising the device as in any one of claims 1-9, wherein said device is loaded into a catheter (103).

11. The system of claim 10, wherein said catheter (103) further comprises a guide wire (102).

12. The system as in claim 10 or claim 11, wherein said distal end of said system comprises a protected lip.

13. The system as in any one of claims 10-12, wherein said protected lip further comprises a dilator (101); and/or wherein said dilator (101) is positioned distal to said catheter (103) and has a smaller diameter than said catheter (103); and/or wherein said lateral structure (200) and filter (201) of said device (100) is compressed within said catheter (103) and positioned behind said dilator (101).

14. The system as in any one of claims 10-13, wherein said lateral structure (200) and filter (201) of said device (100) expands upon the retraction of said catheter (103) relative to said lateral structure (200) and filter (201) of said device (100).

15. The system as in any one of claims 10-14 further comprising a pigtail catheter (104); and/or wherein the opening of said catheter (103) is not perpendicular to the longitudinal axis of said catheter (103).

## Patentansprüche

1. Vorrichtung zur Verhinderung des Eindringens von Emboli in sekundäre Gefäße durch Umlenken, wobei die Vorrichtung umfasst: eine laterale Struktur (200),
die aus einem Federringdraht besteht und eine längliche Form aufweist;
einen im Wesentlichen ebenen, länglichen Filter (201), der entlang der Länge und Breite der lateralen Struktur (200) befestigt ist; wobei die laterale Struktur so dimensioniert ist, dass bei Einbringung der Vorrichtung in einem Aortenbogen eines menschlichen Subjekts, die Vorrichtung die oberen Wände des Aortenbogens berührt;
**dadurch gekennzeichnet, dass**:
ein erster Draht (202) am distalen Ende der lateralen Struktur befestigt ist; und ein zweiter Draht (203) am proximalen Ende der lateralen Struktur befestigt ist,
wobei der erste und der zweite Draht zum Zuführen der Vorrichtung zum Aortenbogen konfiguriert sind.

2. Vorrichtung nach Anspruch 1, wobei der erste Draht (202) ferner einen schraubenartigen Mechanismus (401) umfasst, der an seinem distalen Ende positioniert ist, und der Filter (201) oder die laterale Struktur (200) eine passende Komponente an seinem distalen Ende umfasst, die geeignet ist, den schraubenartigen Mechanismus (401) aufzunehmen.

3. Vorrichtung nach Anspruch 2, wobei die passende Komponente einen Wulst (400) umfasst, der am distalen Ende des Filters (201) oder der lateralen Struktur (200) befestigt ist.

4. Vorrichtung nach einem der Ansprüche 1 bis 3, wobei sich der Filter (201) über den von der lateralen Struktur (200) umschriebenen Umfang hinaus erstreckt; oder wobei der erste Draht (202) steifer als der zweite Draht (203) ist, oder der zweite Draht (203) steifer als der erste Draht (202) ist; oder wobei der erste Draht (202) und der zweite Draht (203) die gleiche Steifigkeit aufweisen.

5. Vorrichtung nach einem der Ansprüche 1 bis 4, wobei die Vorrichtung aus Metall- oder Polymerdraht hergestellt ist; oder wobei die Vorrichtung aus Nitinoldraht hergestellt ist.

6. Vorrichtung nach einem der Ansprüche 1 bis 5, wobei der Durchmesser des Drahtes etwa 1,0 mm oder weniger beträgt.

7. Vorrichtung nach Anspruch 6, wobei die laterale Struktur (200) zwei oder mehr Windungen des Drahtes mit einem Durchmesser von weniger als etwa 1,0 mm umfasst.

8. Vorrichtung nach einem der Ansprüche 1 bis 7, wobei die Vorrichtung außerdem röntgendichte Markierungen umfasst; und/oder wobei der Filter (201) für Emboli mit einer Abmessung von mehr als 50, 100 oder 200 µm nicht wesentlich durchlässig ist; und/oder wobei der erste (202) oder zweite (203) Draht einen leicht verformbaren Draht umfasst.

9. Vorrichtung nach einem der Ansprüche 1 bis 8, wobei der zweite Draht (203) ein Hohlrohr (600) ist; und/oder wobei die laterale Struktur (200) ferner zwei Kugeln oder Spitzen (700) an den distalen und proximalen Enden umfasst; und/oder wobei der erste Draht (202) und/oder der zweite Draht (202) eine Schlinge (701) umfasst, und das proximale und/oder distale Ende des Filters (201) einen Wulst (400) oder ein anderes Aufnahmeelement umfasst, das mit der Schlinge (701) verbunden ist; und/oder wobei der Filter (201) ferner ein Hohlrohr (600) umfasst, das entlang der Länge des Filters positioniert ist.

10. System, das die Vorrichtung nach einem der Ansprüche 1 bis 9 umfasst, wobei die Vorrichtung in einen Katheter (103) eingebracht ist.

11. System nach Anspruch 10, wobei der Katheter (103) ferner einen Führungsdraht (102) umfasst.

12. System nach Anspruch 10 oder Anspruch 11, wobei das distale Ende des Systems eine geschützte Lippe umfasst.

13. System nach einem der Ansprüche 10 bis 12, wobei die geschützte Lippe ferner einen Dilatator (101) umfasst; und/oder wobei der Dilatator (101) distal zum Katheter (103) positioniert ist und einen kleineren Durchmesser als der Katheter (103) aufweist; und/oder wobei die laterale Struktur (200) und der Filter (201) der Vorrichtung (100) innerhalb des Katheters (103) komprimiert und hinter dem Dilatator (101) positioniert sind.

14. System nach einem der Ansprüche 10 bis 13, wobei sich die laterale Struktur (200) und der Filter (201) der Vorrichtung (100) beim Zurückziehen des Katheters (103) relativ zur lateralen Struktur (200) und dem Filter (201) der Vorrichtung (100) ausdehnen.

15. System nach einem der Ansprüche 10 bis 14, weiterhin umfassend einen Pigtail-Katheter (104); und/oder wobei die Öffnung des Katheters (103) nicht senkrecht zur Längsachse des Katheters (103) verläuft.

## Revendications

1. Dispositif destiné à empêcher les emboles de pénétrer dans les vaisseaux secondaires, comprenant : une structure latérale (200) constituée d'un fil à anneau élastique de forme allongée ;
un filtre allongé, sensiblement plan (201), fixé à la longueur et à la largeur de ladite structure latérale (200) ; dans lequel ladite structure latérale est dimensionnée de telle sorte que lors de l'installation dudit dispositif dans une crosse aortique d'un sujet humain, ledit dispositif entre en contact avec les parois supérieures dudit arc aortique ;
**caractérisé par** :
un premier fil (202) fixé à l'extrémité distale de ladite structure latérale ; et un deuxième fil (203) fixé à l'extrémité proximale de ladite structure latérale
dans lequel lesdits premier et deuxième fils sont configurés pour amener ledit dispositif audit arc aortique.

2. Dispositif selon la revendication 1, dans laquelle ledit premier fil (202) comprend en outre un mécanisme en forme de vis (401) positionné à son extrémité distale et ledit filtre (201) ou ladite structure latérale (200) comprend un composant d'accouplement à son extrémité distale adapté pour recevoir ledit mécanisme en forme de vis (401).

3. Dispositif selon la revendication 2, dans laquelle ledit composant d'accouplement comprend une bille (400) fixé à l'extrémité distale dudit filtre (201) ou de ladite structure latérale (200).

4. Dispositif selon l'une quelconque des revendications 1 à 3, dans lequel ledit filtre (201) s'étend au-delà du périmètre circonscrit par ladite structure latérale (200) ; ou dans lequel ledit premier fil (202) est plus rigide que ledit deuxième fil (203), ou ledit deuxième fil (203) est plus rigide que ledit premier fil (202) ; ou dans lequel ledit premier fil (202) et ledit deuxième fil (203) sont de rigidité égale.

5. Dispositif selon l'une quelconque des revendications 1 à 4, dans lequel ledit dispositif est fabriqué à partir d'un fil métallique ou polymère ; ou dans lequel ledit dispositif est fabriqué à partir de fil de Nitinol.

6. Dispositif selon l'une quelconque des revendications 1 à 5, dans lequel le diamètre dudit fil est d'environ 1,0 mm ou moins.

7. Dispositif selon la revendication 6, dans lequel ladite structure latérale (200) comprend deux ou plusieurs torsions dudit fil ayant un diamètre inférieur à environ 1,0 mm.

8. Dispositif selon l'une quelconque des revendications 1 à 7, dans lequel ledit dispositif comprend en outre des marqueurs ; et/ou dans lequel ledit filtre (201) n'est pas significativement perméable aux emboles ayant une dimension de supérieure à 50, 100 ou 200 µm ; et/ou dans lequel ledit premier (202) ou deuxième (203) fil comprend un fil facilement malléable.

9. Dispositif selon l'une quelconque des revendications 1 à 8, dans lequel ledit deuxième fil (203) est un tube creux (600) ; et/ou dans lequel ladite structure latérale (200) comprend en outre deux billes ou pointes (700) auxdites extrémités distale et proximale ; et/ou dans lequel ledit premier fil (202) et/ou ledit premier fil (202) comprennent un collet (701), et ladite extrémité proximale et/ou distale dudit filtre (201) comprend une bille (400), ou un autre élément de réception, élément qui se joint audit collet (701) ; et/ou dans lequel ledit filtre (201) comprend en outre un tube creux (600) positionné sur la longueur dudit filtre.

10. Système comprenant le dispositif selon l'une quelconque des revendications 1 à 9, dans lequel ledit dispositif est chargé dans un cathéter (103).

11. Système selon la revendication 10, dans lequel ledit cathéter (103) comprend en outre un fil de guidage (102).

12. Système selon la revendication 10 ou la revendication 11, dans lequel ladite extrémité distale dudit système comprend une lèvre protégée.

13. Système selon l'une quelconque des revendications 10 à 12, dans lequel ladite lèvre protégée comprend en outre un dilatateur (101) ; et/ou dans lequel ledit dilatateur (101) est positionné de manière distale par rapport audit cathéter (103) et a un diamètre plus petit que ledit cathéter (103) ; et/ou dans lequel ladite structure latérale (200) et le filtre (201) dudit dispositif (100) sont comprimés à l'intérieur dudit cathéter (103) et positionnés derrière ledit dilatateur (101).

14. Système selon l'une quelconque des revendications 10 à 13, dans lequel ladite structure latérale (200) et le filtre (201) dudit dispositif (100) se dilatent lors de la rétraction dudit cathéter (103) par rapport à ladite structure latérale (200) et au filtre (201) dudit dispositif (100).

15. Système selon l'une quelconque des revendications 10 à 14, comprenant en outre un cathéter en queue de cochon (104) ; et/ou dans lequel l'ouverture dudit cathéter (103) n'est pas perpendiculaire à l'axe longitudinal dudit cathéter (103).
